# EUROPEAN PATENT APPLICATION

(11) **EP 3 560 506 A1**
(43) Date of publication of application: **30.10.2019**
(21) Application number: 17883777.9
(22) Date of filing: 20.12.2017
(51) Int. Cl.: A61K 36/70, A23L 33/105

(54) **PHARMACEUTICAL COMPOSITION COMPRISING INDIGO PULVERATA LEVIS EXTRACT OR FRACTION THEREOF AS EFFECTIVE INGREDIENT FOR PREVENTING OR TREATING INFLAMMATORY BOWEL DISEASE**

(30) Priority: 20.12.2016 KR 20160174921
(71) Applicant: Seoul National University Hospital, Seoul 03080 (KR); Dong-A ST Co., Ltd., Dongdaemun-gu Seoul 02587 (KR)
(72) Inventor: CHO, Young Woong, Suwon-si Gyeonggi-do 16690 (KR); SON, Mi Won, Yongin-si Gyeonggi-do 17073 (KR); CHOI, Sang Zin, Suwon-si Gyeonggi-do 16709 (KR); CHOI, Song Hyen, Suwon-si Gyeonggi-do 16528 (KR); JEONG, Jin Seok, Seoul 06549 (KR); SONG, In Sung, Seoul 05510 (KR); KIM, Nayoung, Seongnam-si Gyeonggi-do 13588 (KR)
(74) Representative: HGF Limited
(86) International application number: PCT/KR2017/015162
(87) International publication number: WO 2018/117659

(57) **Abstract**

The present invention relates to a pharmaceutical composition comprising an Indigo Pulverata Levis extract or a fraction thereof as an effective ingredient for preventing or treating inflammatory bowel disease. Exhibiting the effect of reducing the activity and expression of inflammatory activation markers and alleviating the disease activity index of inflammatory bowel disease, an Indigo Pulverata Levis extract or a fraction thereof according to the present invention may be useful as an agent for preventing and treating inflammatory bowel disease.

## Description

### Technical Field

The present invention relates to a composition comprising an Indigo Pulverata Levis extract or a fraction thereof as an effective ingredient for preventing or treating inflammatory bowel disease.

### Background

Inflammatory bowel disease (IBD) is a common name for chronic relapsing inflammatory diseases caused by a dysregulation of immune systems in small and large intestines. In case of the inflammatory bowel disease represented by Crohn's disease (CD) and ulcerative colitis (UC), it is known that the dysregulation of immune reactions may be a key cause of such disease (Braus et al., Clin Immunol, 2009, 132, 1-9; McGuckin et al., Inflamm Bowel Dis, 2008, 15, 100-113).

Inflammatory bowel disease used to be recognized as a rare disease in the Asian region. However, with a recent westernization of living habits, the number of patients with inflammatory bowel disease has tended to increase rapidly since 1980's in the East, too. Accordingly, there has been active study on alleviating inflammatory bowel disease. Recently, some cytokines or cells have been found to be associated with inflammatory bowel disease, thus resulting in a development of numerous biological agents for selectively attacking certain molecules or paths connected to intestinal inflammations (Sandborn et al., Gastroenterology, 2002, 122: 521-530).

Now, as a therapeutic agent for inflammatory bowel disease, 5-aminosalicylic acid (5-ASA)-based drugs for blocking a production of prostaglandins, for example, sulfalazine, etc., have been used, or steroid immunosuppressive drugs have been used.

However, sulfalazine may cause side effects such as abdominal fullness, headache, rash, liver disease, leukopenia, agranulocytosis, male infertility, etc. Also, the steroid immunosuppressive drugs have their limits in that such drugs may not improve a long-term prognosis and should be used only in acute cases, due to side effects such as induced infectious diseases, secondary adrenocortical insufficiency, peptic ulcers, diabetes, mental disorders, etc. Thus, there is still a need for developing a therapeutic agent for inflammatory bowel disease without a side effect.

Meanwhile, Indigo Pulverata Levis, which is an annual herbaceous plant growing naturally in Korea, China and Japan, refers to the powder obtained by fermenting the leaves of *Persicaria tinctoria* H. Gross or *Baphicacanthus cusia* Bremek., of which plant height is 60-100 cm or so.

From old times, Indigo Pulverata Levis has been known in oriental medicine to have various efficacies, such as fever removal and detoxification, extermination of insects, blood purification and removal of boils, etc., in particular. However, nothing has been known about its working effects, etc., on inflammatory bowel disease through clinical relief of symptoms such as inhibition of inflammatory factors, jelly stool, bloody stool, diarrhea, etc.

Accordingly, as a result of having made efforts to develop a therapeutic agent for inflammatory bowel disease from medicinal plants, the present inventors have identified that an Indigo Pulverata Levis extract or a fraction thereof exhibits an effect of reducing an inflammation score and the fraction shows a remarkable effect, in particular. Also, as said extract or the fraction thereof significantly reduces the disease activity index (DAI) of inflammatory bowel disease in an in vivo model for inflammatory bowel disease, the present inventors have identified that the Indigo Pulverata Levis extract or the fraction thereof may be valuably used as an effective ingredient of a pharmaceutical composition for treating and preventing inflammatory bowel disease, thereby completing the present invention.

### [Prior Art Reference]

### [Non-Patent Document]

(Non-Patent Document 1) Braus et al., Clin Immunol, 2009, 132, 1-9
(Non-Patent Document 2) McGuckin et al., Inflamm Bowel Dis, 2008, 15, 100-113
(Non-Patent Document 3) Sandborn et al., Gastroenterology, 2002, 122: 521-530

### Detailed Description of the Invention

### Technical Problem

An objective of the present invention is to provide a pharmaceutical composition containing an Indigo Pulverata Levis extract or a fraction thereof as an effective ingredient for preventing or treating inflammatory bowel disease.

Other objective of the present invention is to provide a health functional food containing an Indigo Pulverata Levis extract or a fraction thereof as an effective ingredient for preventing or alleviating inflammatory bowel disease.

Another objective of the present invention is to provide a method for preventing or treating inflammatory bowel disease, including a step of administering a pharmaceutical composition containing an Indigo Pulverata Levis extract or a fraction thereof as an effective ingredient into subjects in need.

Also, another objective of the present invention is to provide an intended use of an Indigo Pulverata Levis extract or a fraction thereof for preventing or treating inflammatory bowel disease.

Further, another objective of the present invention is to provide a use of an Indigo Pulverata Levis extract or a fraction thereof for producing a drug having an effect of preventing or treating inflammatory bowel disease.

### Technical Solution

As a result of having studied to develop a natural product medicine having an effect of preventing or treating inflammatory bowel disease, the present inventors have identified that an Indigo Pulverata Levis extract or a fraction thereof exhibits an excellent effect of preventing and treating inflammatory bowel disease, thereby completing the present invention.

In one aspect for achieving said objectives, the present invention provides a pharmaceutical composition containing an Indigo Pulverata Levis extract or a fraction thereof as an effective ingredient for preventing or treating inflammatory bowel disease.

In the present invention, the term "inflammatory bowel disease" collectively refers to the diseases of causing inflammation in intestinal tracts, and particularly may be the chronic relapsing inflammatory diseases caused by a dysregulation of immune systems in small and large intestines. Said inflammatory bowel disease includes ulcerative colitis and Crohn's disease.

The Indigo Pulverata Levis used in preparing an extract of the present invention or a fraction thereof may be purchased from among commercial products, or may be directly prepared and used.

The Indigo Pulverata Levis extract of the present invention may include a distilled water or organic solvent extract all, of which volume is 1 to 30 times more than a dry weight of Indigo Pulverata Levis, and particularly may be an organic solvent extract, a crude extract or a concentrate thereof.

Said organic solvent may be at least one selected from the group consisting of lower alcohol having 1 to 4 carbon atoms, hexane, ethyl acetate, dichloromethane, ether, chloroform and acetone. Said lower alcohol having 1 to 4 carbon atoms may be at least one selected from the group consisting of methanol, ethanol, propanol, isopropanol, butanol and n-butanol. The lower alcohol having 1 to 4 carbon atoms may include anhydrous or hydrous alcohol all. Said alcohol, for example, ethanol may be 1 to 100% (v/v%), particularly 30 to 100%, more particularly 30 to 80%, and much more particularly 30%, 35%, 40%, 45%, 50%, 55%, 60%, 65% or 70% alcohol.

In the present invention, the Indigo Pulverata Levis extract may be extracted with water, lower alcohol having 1 to 4 carbon atoms or mixtures thereof as solvent, and said lower alcohol having 1 to 4 carbon atoms may be ethanol, methanol or butanol. According to one embodiment aspect of the present invention, the Indigo Pulverata Levis extract may be an extract, which is obtained with 70% ethanol.

The fraction of the present invention may be an organic solvent fraction, and said organic solvent may be at least one selected from the group consisting of hexane, chloroform, ethyl acetate and butanol.

In the present invention, the fraction of the Indigo Pulverata Levis extract may be a hexane fraction, a chloroform fraction, an ethyl acetate fraction or a butanol fraction, which is obtained sequentially by concentrating the Indigo Pulverata Levis extract, and then by adding organic solvent, particularly hexane, chloroform, ethyl acetate and butanol thereto, again.

According to one embodiment aspect of the present invention, the fraction of the Indigo Pulverata Levis extract may be prepared into the hexane fraction of the ethanol extract of Indigo Pulverata Levis, the chloroform fraction thereof, the ethyl acetate fraction thereof and the butanol fraction thereof by carrying out an extraction for Indigo Pulverata Levis with ethanol to obtain an ethanol extract therefrom; then by adding water to said ethanol extract and fractionating the resulting mixture with hexane to separate a hexane fraction layer and a water layer from each other firstly; then by fractionating said water layer with chloroform to separate a chloroform fraction layer and a water layer from each other secondly; then by fractionating said water layer with ethyl acetate to separate an ethyl acetate fraction layer and a water layer from each other thirdly; and then by fractionating said water layer with butanol to separate a butanol fraction layer therefrom lastly.

The Indigo Pulverata Levis extract or the fraction thereof according to the present invention may be prepared into a powder state by using a conventional drying method, such as low pressure drying, spray drying, freeze drying or the like to remove remaining lower alcohol and organic solvent therefrom so that such extract or the fraction thereof may be suitable to be used as a raw material for medicine. Particularly, said Indigo Pulverata Levis extract may be a concentrated liquid or a freeze-dried powder form. According to one embodiment aspect of the present invention, the Indigo Pulverata Levis extract may be a soft extract, which is a concentrated liquid form.

In one exemplary embodiment of the present invention, as a result of administering an Indigo Pulverata Levis extract or a fraction thereof into an animal model with induced inflammatory bowel disease, it was identified that there is an effect of increasing a colon length, reducing the activity and expression of inflammatory activation markers, and improving the disease activity index of inflammatory bowel disease, and thus it might be seen that said Indigo Pulverata Levis extract is effective in treating and preventing inflammatory bowel disease. Particularly, it might be seen that an ethyl acetate fraction of the Indigo Pulverata Levis extract exhibits the most excellent effect.

The pharmaceutical composition of the present invention may be formulated into a dosage form for oral administration, for example, tablets, troches, lozenges, watersoluble or oil suspensions, prepared powders or granules, emulsions, hard or soft capsules, syrups, elixirs or the like according to a conventional method for preventing or treating inflammatory bowel disease.

To formulate into the dosage form such as the tablets, capsules and the like, the followings may be contained: binders such as lactose, saccharose, sorbitol, mannitol, starch, amylopectin, cellulose or gelatin; excipients such as dicalcium phosphate; disintegrants such as maize starch or sweet potato starch; or lubricants such as magnesium stearate, calcium stearate, sodium stearyl fumarate or polyethylene glycol wax. In case of the capsule dosage form, liquid carriers such as fatty oil may be contained in addition to the above-mentioned materials.

Also, the pharmaceutical composition of the present invention may be parenterally administered. The parenteral administration may be performed by means of subcutaneous injection, intravenous injection, intramuscular injection or intrathoracic injection method. To formulate into the dosage form for parenteral administration, said composition may be prepared into solution by being mixed in water with stabilizers or buffer agents, and then may be formulated again into a unit form for administration of ampoule or vial.

A dosage of the pharmaceutical composition according to the present invention needs to be a pharmaceutically effective amount. The "pharmaceutically effective amount" means an amount enough to prevent or treat diseases at a reasonable benefit/risk ratio applicable to medical treatment, and a level of effective dose may be variously selected by those skilled in the art according to factors such as a formulation method, a patient's condition, weight, gender and age, a degree of disease, a drug form, an administration route and period, an excretion rate, reaction sensitivity, etc. The effective amount may vary depending on a route of treatment, a use of excipients and a possibility of being used with other drugs, as recognized by those skilled in the art.

A dosage or dose of the pharmaceutical composition containing the Indigo Pulverata Levis extract or the fraction thereof according to the present invention as an effective ingredient may be diversified according to a patient's age, physical condition, body weight, etc., but may be preferably administered within a range of 10 to 100 mg/kg (body weight)/day in general. And such administration may be performed once a day or divided into several times a day within the range of daily effective inputs.

In another aspect, the present invention provides a health functional food containing an Indigo Pulverata Levis extract or a fraction thereof as an effective ingredient for preventing or alleviating inflammatory bowel disease.

The Indigo Pulverata Levis extract or the fraction thereof according to the present invention may be contained in the food, and thus may effectively prevent or alleviate inflammatory bowel disease upon its intake.

The Indigo Pulverata Levis extract or the fraction thereof according to the present invention may be added as it is or may be used along with other foods or food ingredients, and may be appropriately used according to a conventional method.

A type of said food is not particularly limited. As an example of food, to which said Indigo Pulverata Levis extract or the fraction thereof may be added, there are meats, sausages, breads, chocolates, candies, snacks, confectioneries, pizzas, instant noodles, other noodles, chewing gums, dairy products including ice creams, various types of soup, beverages, teas, health drinks, alcohol beverages, vitamin complexes and the like, and all the health functional foods are included in a conventional sense. Besides the foods mentioned above, the Indigo Pulverata Levis extract or the fraction thereof according to the present invention may contain various nutritional supplements, vitamins, electrolytes, flavoring agents, coloring agents, pectic acid and salts thereof, alginic acid and salts thereof, organic acid, protective colloidal thickeners, pH adjusting agents, stabilizers, preservatives, glycerin, alcohol, carbonation agents used in carbonated beverage, etc. Besides, the Indigo Pulverata Levis extract or the fraction thereof according to the present invention may contain natural fruit juice or pulp for preparing fruit juice beverage and vegetable based beverage. Such ingredients may be used independently or in combination.

Also, in another aspect, the present invention provides a method for preventing or treating inflammatory bowel disease, including a step of administering a pharmaceutical composition containing an Indigo Pulverata Levis extract or a fraction thereof as an effective ingredient into subjects in need.

As used herein, the term "Indigo Pulverata Levis," "Indigo Pulverata Levis extract," "fraction," "bowel disease," "administration" and the like are the same as described above.

In the present invention, said subjects refer to animals, and may be typically mammals, on which treatment using the extract of the present invention may exhibit a beneficial effect. A preferable example of such subjects may include primates like humans. Also, such subjects may include all the subjects having a symptom of inflammatory bowel disease, or having a risk of developing such symptom.

Further, in another aspect, the present invention provides an intended use of using an Indigo Pulverata Levis extract or a fraction thereof for preventing or treating inflammatory bowel disease.

Furthermore, in another aspect, the present invention provides a use of an Indigo Pulverata Levis extract or a fraction thereof for producing a drug having an effect of preventing or treating inflammatory bowel disease.

### Advantageous Effects

Exhibiting the effect of reducing the activity and expression of inflammatory activation markers and alleviating the disease activity index of inflammatory bowel disease, an Indigo Pulverata Levis extract or a fraction thereof according to the present invention may be valuably used as an effective ingredient of a pharmaceutical composition for preventing and treating inflammatory bowel disease.

### Brief Description of the Drawings

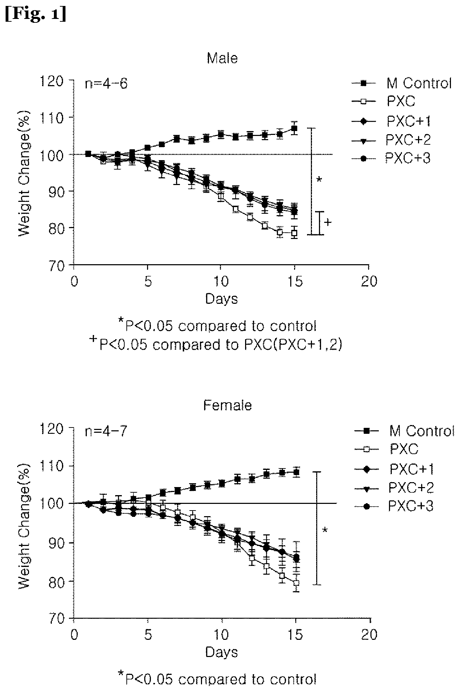

Fig. 1 is a graph of showing a change in weights as a result of administering an Indigo Pulverata Levis extract or a fraction thereof into models with piroxicam-induced bowel disease:
   PXC is a group dosed with piroxicam; PXC+1 is a group dosed with piroxicam + 70% ethanol extract of Indigo Pulverata Levis; PXC+2 is a group dosed with piroxicam + ethyl acetate fraction of 70% ethanol extract of Indigo Pulverata Levis; and PXC+3 is a group dosed with piroxicam + butanol fraction of 70% ethanol extract of Indigo Pulverata Levis: * indicates a remarkable difference from a normal group, while † does a remarkable difference from a group with induced disease, which are hereinafter the same.
Fig. 2 is an image of showing a change in colon lengths as a result of administering an Indigo Pulverata Levis extract or a fraction thereof into models with piroxicam-induced bowel disease.
Fig. 3 is a graph of comparing colon lengths with each other as a result of administering an Indigo Pulverata Levis extract or a fraction thereof into models with piroxicam-induced bowel disease.
Fig. 4 is a graph of showing an inhibition in MPO expressions in a bowel tissue as a result of administering an Indigo Pulverata Levis extract or a fraction thereof into models with piroxicam-induced bowel disease.
Fig. 5 is a graph of showing an inhibition in IL-6 expressions in a bowel tissue as a result of administering an Indigo Pulverata Levis extract or a fraction thereof into models with piroxicam-induced bowel disease.
Fig. 6 is a graph of showing an inhibition in IL-1b expressions in a bowel tissue as a result of administering an Indigo Pulverata Levis extract or a fraction thereof into models with piroxicam-induced bowel disease.
Fig. 7 is a graph of showing an inhibition in IL-17 expressions in a bowel tissue as a result of administering an Indigo Pulverata Levis extract or a fraction thereof into models with piroxicam-induced bowel disease.
Fig. 8 is a graph of showing a decrease in the disease activity index (DAI) of bowel disease as a result of administering an ethyl acetate fraction of 70% ethanol extract of Indigo Pulverata Levis into models with piroxicam-induced bowel disease.
Fig. 9 is a graph of showing an increase in survival rates as a result of administering an ethyl acetate fraction of 70% ethanol extract of Indigo Pulverata Levis into models with piroxicam-induced bowel disease.
   Statistical analysis based on Log-rank (Mantel-Cox) test.
Fig. 10 is an image of identifying a decrease in pathological cell destruction in a bowel tissue with a microscope as a result of administering an ethyl acetate fraction of 70% ethanol extract of Indigo Pulverata Levis into models with piroxicam-induced bowel disease.
Fig. 11 is a graph of showing a score of pathological cell destruction in a bowel tissue as a result of administering an ethyl acetate fraction of 70% ethanol extract of Indigo Pulverata Levis into models with piroxicam-induced bowel disease.
Fig. 12 is a graph of showing a change in weights as a result of administering an Indigo Pulverata Levis extract or a fraction thereof into models with dextran sulfate sodium (DSS)-induced bowel inflammation:
   DSS is a group dosed with dextran sulfate sodium; DSS+1 is a group dosed with dextran sulfate sodium + 70% ethanol extract of Indigo Pulverata Levis; DSS+2 is a group dosed with dextran sulfate sodium + ethyl acetate fraction of 70% ethanol extract of Indigo Pulverata Levis; and DSS+3 is a group dosed with dextran sulfate sodium + butanol fraction of 70% ethanol extract of Indigo Pulverata Levis: * indicates a remarkable difference from a normal group, while † does a remarkable difference from a group with induced disease, which are hereinafter the same.

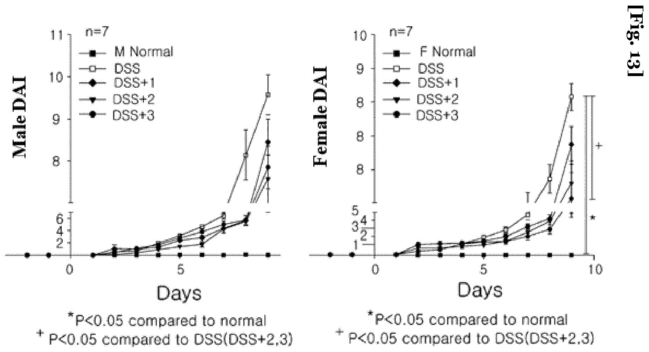

Fig. 13 is a graph of showing a decrease in the disease activity index (DAI) of bowel inflammation as a result of administering an Indigo Pulverata Levis extract or a fraction thereof into models with dextran sulfate sodium (DSS)-induced bowel inflammation.

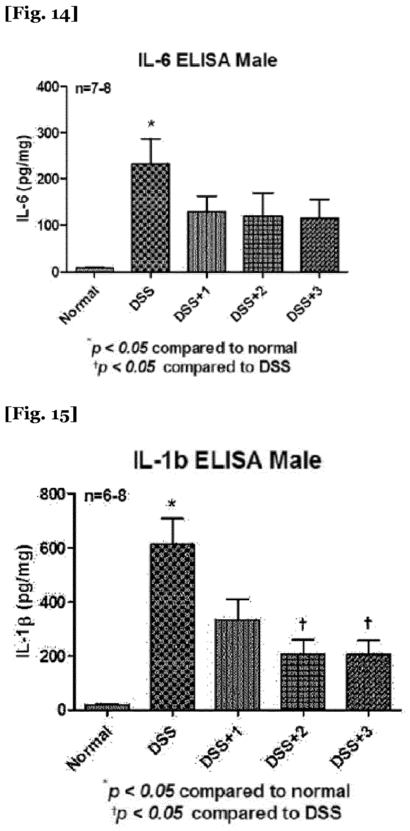

Fig. 14 is a graph of showing an inhibition in IL-6 expressions in a bowel tissue as a result of administering an Indigo Pulverata Levis extract or a fraction thereof into models with dextran sulfate sodium (DSS)-induced bowel inflammation.
Fig. 15 is a graph of showing an inhibition in IL-1b expressions in a bowel tissue as a result of administering an Indigo Pulverata Levis extract or a fraction thereof into models with dextran sulfate sodium (DSS)-induced bowel inflammation.

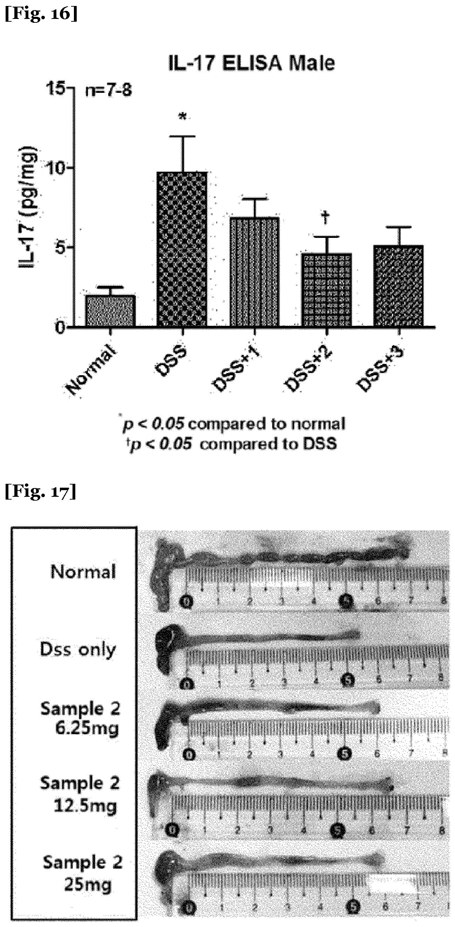

Fig. 16 is a graph of showing an inhibition in IL-17 expressions in a bowel tissue as a result of administering an Indigo Pulverata Levis extract or a fraction thereof into models with dextran sulfate sodium (DSS)-induced bowel inflammation.
Fig. 17 is an image of showing an increase in colon lengths as a result of administering an ethyl acetate fraction of 70% ethanol extract of Indigo Pulverata Levis into models with dextran sulfate sodium-induced bowel inflammation.

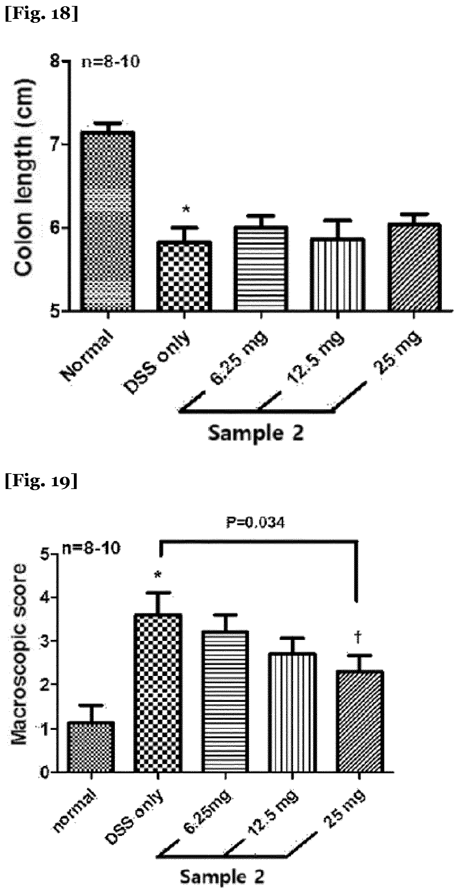

Fig. 18 is a graph of showing an increase in colon lengths as a result of administering an ethyl acetate fraction of 70% ethanol extract of Indigo Pulverata Levis into models with dextran sulfate sodium-induced bowel inflammation.
Fig. 19 is a graph of showing an inhibition in macroscopic scores of inflammations in a colon tissue as a result of administering an Indigo Pulverata Levis extract into models with dextran sulfate sodium-induced bowel inflammation.

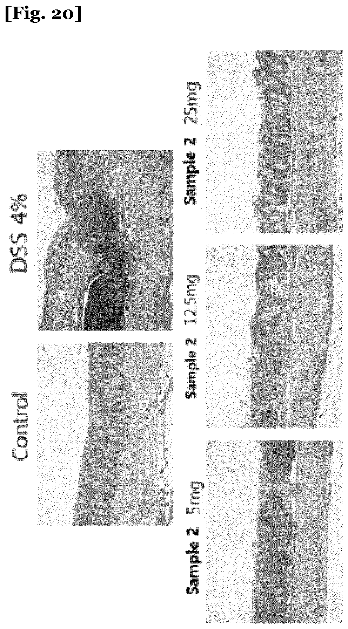

Fig. 20 is an image of identifying a decrease in pathological cell destruction in a bowel tissue with a microscope as a result of administering an ethyl acetate fraction of 70% ethanol extract of Indigo Pulverata Levis into models with dextran sulfate sodium-induced bowel inflammation.

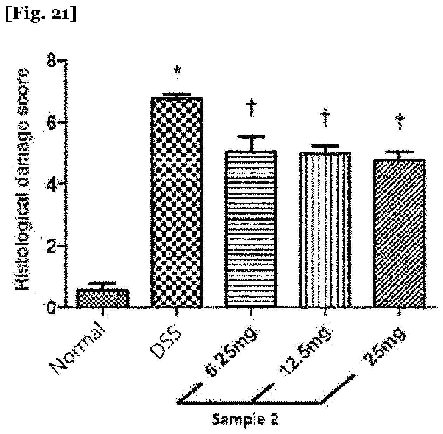

Fig. 21 is a graph of showing a score of pathological cell destruction in a bowel tissue as a result of administering an ethyl acetate fraction of 70% ethanol extract of Indigo Pulverata Levis into amodels with dextran sulfate sodium-induced bowel inflammation.

### Best Mode for Invention

Hereinafter, the configuration and effects of the present invention will be described in more detail through Examples. However, the following Examples are provided only for the purpose of illustrating the present invention, and thus the content of the present invention is not limited thereto.

### Example 1: Preparation for an Indigo Pulverata Levis extract and a fraction thereof

Indigo Pulverata Levis and 70% ethanol were stirred for 48 hours to carry out an extraction, and filtered to evaporate solvent and water therefrom, such that an Indigo Pulverata Levis extract was prepared in a form of soft extract. After that, water was added to said extract, after which hexane was added thereto and fractionated, such that an organic solvent layer and a water layer were separated from each other, firstly. Chloroform (CHCl₃) was added again to said firstly-separated water layer and fractionated, such that an organic solvent layer and a water layer were separated from each other, secondly. Ethyl acetate (EtOAc) was added again to said secondly-separated water layer, such that an organic solvent layer and a water layer were separated from each other, thirdly. Butanol (BuOH) was added again to said thirdly-separated water layer, after which an organic solvent layer and a water layer were separated from each other, fourthly, such that a hexane fraction, a chloroform fraction, an ethyl acetate fraction and a butanol fraction were collected respectively. In the following experimental examples, a 70% ethanol extract of Indigo Pulverata Levis, an ethyl acetate fraction thereof and a butanol fraction thereof were used as samples as shown in a following table 1.

**[Table 1]**

| Sample No. | Sample Types |
|---|---|
| 1 | 70% ethanol extract of Indigo Pulverata Levis |
| 2 | Ethyl acetate fraction of the 70% ethanol extract of Indigo Pulverata Levis |
| 3 | Butanol fraction of the 70% ethanol extract of Indigo Pulverata Levis |

### Experimental Example 1: Experiment on an Indigo Pulverata Levis extract for preventing and treating colitis bowel disease

An effect of an Indigo Pulverata Levis extract on preventing and treating inflammatory bowel disease was identified by using animal models for colitis bowel disease. The animal models for colitis bowel disease, which were used in the experiment, were prepared by treating IL-10 knockout mice with piroxicam, wherein such mice were known to be used to identify an effect of preventing and treating bowel disease (Holgersen et al, J Crohns Colitis, 2014;8;147-60; Hale et al. Inflamm Bowel Dis 2005;11:1060-9).

Particularly, while the IL-10-deficient laboratory mice were fed a diet mixed with piroxicam, an Indigo Pulverata Levis extract or a fraction thereof, which had been prepared according to Example 1 above, was orally administered into each of the mice by 25 mg once a day for 13 days as shown in a table 1. A body weight was measured every day for an experiment period of 13 days. After the 14th day of the experiment, the laboratory mice were sacrificed, after which a length of colons was measured and tissues were obtained to measure immunoreactivity factors with an ELISA kit.

As a result of comparing weights, in case of a normal group, a certain level of weights was maintained. However, in case of a group dosed with piroxicam, the weights were drastically decreased. However, in case of a group dosed with the Indigo Pulverata Levis extract and the fraction thereof, it was identified that a rate of decrease in weights was low compared to a group dosed with piroxicam only (Fig. 1).

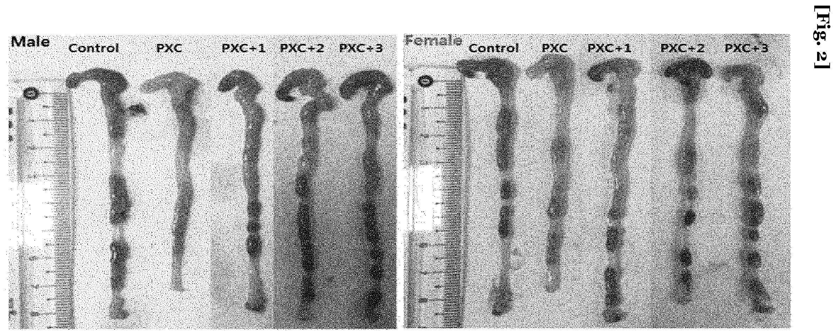

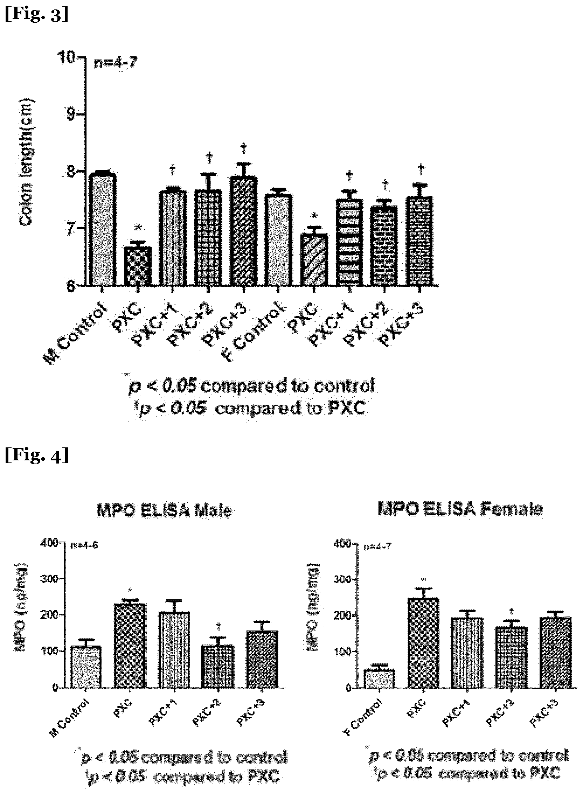

Also, as a result of identifying a change in colon lengths, it was shown that the length of colons was decreased in a case of a group dosed with piroxicam-induced colitis bowel disease. However, in case of the group dosed with the Indigo Pulverata Levis extract and the fraction thereof, it was identified that the length of colons was increased compared to the group dosed with piroxicam only and was recovered up to a level of the normal group (Figs. 2 and 3).

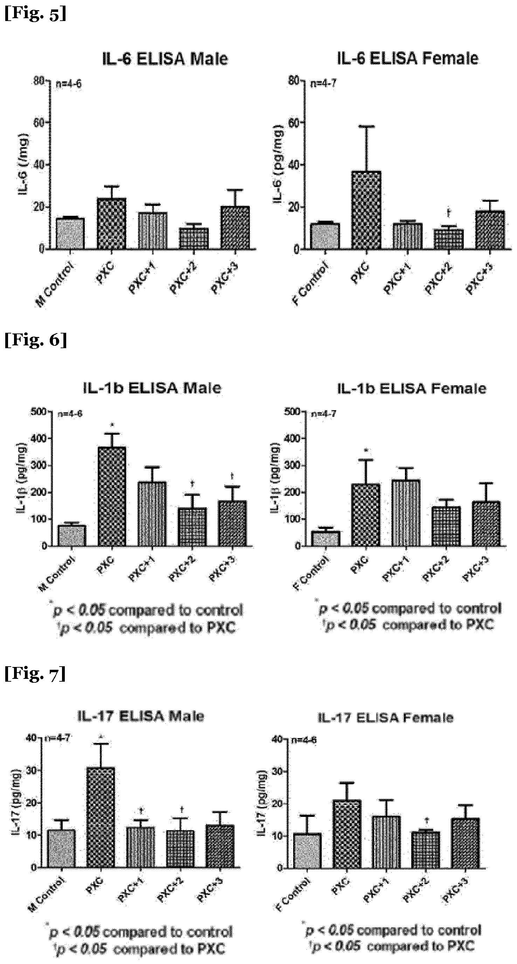

Furthermore, as a result of comparing the levels of immune factors associated with colitis disease, it was shown that the level of MPO, IL-6, IL-1b and IL-17 was increased in case of the group dosed with piroxicam-induced colitis bowel disease. However, in case of the group dosed with the Indigo Pulverata Levis extract and the fraction thereof, it was shown that said level of immune factors was decreased compared to the group dosed with piroxicam only (Figs. 4 to 7).

Meanwhile, for the group dosed with an ethyl acetate fraction of the Indigo Pulverata Levis extract, its weights, stool forms and bloody stools were observed during the experiment period of 13 days to measure the disease activity index (DAI) of colitis bowel disease according to a method of a table 2.

**[Table 2]**

| Decrease in Weights | Score | Stool Form | Score | Bloody Stool | Score |
|---|---|---|---|---|---|
| <1% | 0 | Hard and firm | 0 | None | 0 |
| 1-5% | 1 | Firm, but sticky | 1 | Hidden | 1 |
| 5-10% | 2 | Soft, but form maintained | 2 | Visible | 2 |
| 10-15% | 3 | Soft and form lost | 3 | Rectal bleeding | 3 |
| 15-20% | 4 | Liquid with no form | 4 | - | - |

Also, after the 14th day of the experiment, the laboratory mice were sacrificed to separate colons therefrom, after which the presence of histological damages to the colons was identified with a microscope and a score of damages to the colons was given according to a method of a table 3.

**[Table 3]**

| Score (Total=11) | Destruction of normal mucosa | Degree and existence of cells | Range of muscular hypertrophy | Presence of crypts | Presence of goblet cells |
|---|---|---|---|---|---|
| 0 | Normal | Normal | Normal | Absent | Absent |
| 1 | Slight damage | Slight level | Slight level | Present | Present |
| 2 | Moderate damage | Moderate level | Moderate level | - | - |
| 3 | Extensive damage | Transmural infiltration | Extensive damage | - | - |

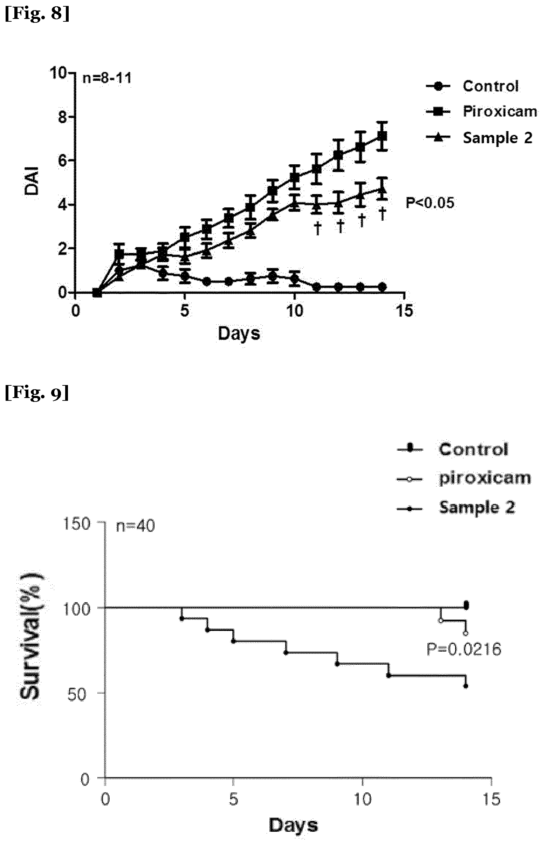

As a result of the experiment, it was identified that the disease activity index (DAI) of bowel disease, reportedly having reflected inflammations best in a colitis model, was significantly alleviated compared with the group dosed with piroxicam-induced colitis (Fig. 8).

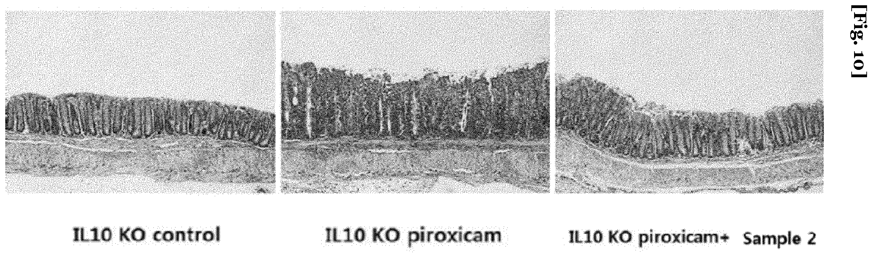

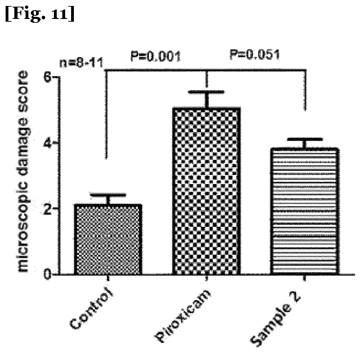

As a result of investigating a survival rate of the animal models during the experiment period, it was identified for the group dosed with the ethyl acetate fraction that the survival rate was high compared to the group dosed with piroxicam to have induced colitis (Fig. 9). Also, it was identified for the group dosed with the ethyl acetate fraction that a degree of damages to colons was decreased to pathologically alleviate colitis (Figs. 10 and 11).

From the experiments above, it was identified that the Indigo Pulverata Levis extract and the fraction thereof had an excellent effect on preventing and treating colitis bowel disease without any toxicity.

### Experimental Example 2: Experiment on an Indigo Pulverata Levis extract for preventing and treating bowel inflammation

An effect of an Indigo Pulverata Levis extract on preventing and treating inflammatory bowel disease was identified by using animal models with dextran sulfate sodium-induced bowel inflammation, which were known to be used to identify an effect of preventing and treating bowel disease (Mizoguchi et al., Prog Mol Biol Transl Sci, 2012:105;263-320; Yan et al., PLos One, 2009:4;e6073).

Particularly, an Indigo Pulverata Levis extract or a fraction thereof, which had been prepared according to Example 1 above, was orally administered into laboratory mice (C57BL6) once a day in an amount suitable for each group as shown in a table 1. In two days after administering a sample, drinking water or drinking water containing 4% dextran sulfate sodium was supplied to such animal models for seven days, which weights, stool forms and bloody stools were observed every day for the experiment period to measure the disease activity index (DAI) of bowel inflammation according to the same standards of the table 2 above as shown in the experimental example 1. And, after the 8th day of the experiment, the laboratory mice were sacrificed, after which colon tissues were obtained therefrom to measure immunoreactivity factors with an ELISA kit.

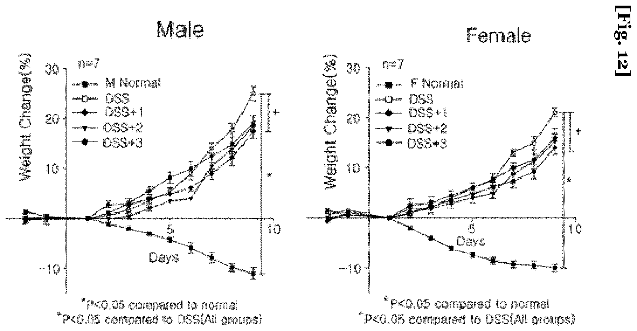

As a result of the experiment, it was identified that weights were slowly increased in a normal group, but drastically decreased in a group dosed with dextran sulfate sodium. However, it was also identified for a group dosed with the Indigo Pulverata Levis extract or the fraction thereof that a degree of decrease in weights was significantly decreased (Fig. 12).

Also, it was identified for the group dosed with dextran sulfate sodium that the disease activity index (DAI) of bowel inflammation was increased to exhibit the characteristics of an inflammatory bowel disease model, but it was also identified for the group dosed with the Indigo Pulverata Levis extract and the fraction thereof that the disease activity index was decreased compared to the group dosed with dextran sulfate sodium (Fig. 13).

Furthermore, as a result of comparing the levels of immune factors associated with inflammatory bowel disease, it was shown that the level of IL-6, IL-1b and IL-17 was increased in a case of the group dosed with dextran sulfate sodium-induced inflammatory bowel disease. However, in case of the group dosed with the Indigo Pulverata Levis extract and the fraction thereof, it was shown that said level of immune factors was decreased compared to the group dosed with dextran sulfate sodium only (Figs. 14 to 16).

Meanwhile, in case of a group dosed with an ethyl acetate fraction of the Indigo Pulverata Levis extract, such fraction was administered at varying concentrations. After the 8th day of the experiment, the laboratory mice were sacrificed, after which a length of colons was measured and a macroscopic score of inflammations in rectum and appendix was measured according to the standards of a table 4 (by using a method for looking into and judging a surface of intestinal mucosa without the use of a microscope). Also, the presence of histological damages to colons was identified with a microscope, and a score of the damages was measured according to a method of a table 5.

**[Table 4]**

| Score | Mucosal Hypertrophy | Ulcer Region | Bleeding of Digestive Organ |
|---|---|---|---|
| 0 | Normal | None | Normal |
| 1 | Slight level | Local bleeding | Slight level |
| 2 | Moderate level | Ulcer (1-3 regions) | Moderate level |
| 3 | - | Ulcer (>3 regions) | - |

**[Table 5]**

| Score (Total 12) | Degree of Mucosal Destruction | Infiltration of Inflammatory Cells | | |
|---|---|---|---|---|
| | | Mucosa | Submucosal Layer | Muscular/Mes enteric Layer |
| 0 | Normal | Normal | Normal | Normal |
| 1 | Hyper-proliferation, irregular crypts and goblet cells | Slight level | Slight-moderate level | Slight-moderate level |
| 2 | Slight or moderate loss of crypts (10-50%) | Moderate level | Moderate level | - |
| 3 | Severe loss of crypts (50-90%) | Moderate level | - | - |
| 4 | Complete loss of crypts and normal epithelial cells | - | - | - |
| 5 | Small or medium ulcers (<10 crypt width) | - | - | - |
| 6 | Large ulcers (≥ 10 crypt width) | - | - | - |

As a result of the experiment, it was identified for the group dosed with dextran sulfate sodium that the length of colons was significantly decreased compared to the normal group, but it was also identified for the group dosed with the ethyl acetate fraction that the length of colons was significantly increased compared to the group dosed with dextran sulfate sodium (Figs. 17 and 18). Also, it was identified for the group dosed with dextran sulfate sodium that a histological score of inflammations was increased to exhibit the characteristics of an inflammatory bowel disease model, but it was also identified for the group dosed with the ethyl acetate fraction that a histological score of inflammations was decreased compared to the group dosed with dextran sulfate sodium (Fig. 19). Also, it was identified for the group dosed with the ethyl acetate fraction that a degree of damages to colons was decreased to pathologically alleviate colitis (Figs. 20 and 21).

From the experiments above, it was identified that the Indigo Pulverata Levis extract and the fraction thereof had an excellent effect on preventing and treating inflammatory bowel disease without any toxicity.

While specific portions of the present invention have been described in detail above, it is apparent to those skilled in the art that such detailed descriptions are set forth to illustrate exemplary embodiments only and are not construed to limit the scope of the present invention.

Thus, it should be understood that the substantial scope of the present invention is defined by the accompanying claims and equivalents thereto.

## Claims

1. A pharmaceutical composition comprising an Indigo Pulverata Levis extract or a fraction thereof as an effective ingredient for preventing or treating inflammatory bowel disease.

2. The pharmaceutical composition for inflammatory bowel disease according to claim 1, wherein the Indigo Pulverata Levis extract is extracted with water, lower alcohol having 1 to 4 carbon atoms or mixtures thereof as solvent.

3. The pharmaceutical composition for inflammatory bowel disease according to claim 2, wherein the lower alcohol is ethanol, methanol or butanol.

4. The pharmaceutical composition for inflammatory bowel disease according to claim 3, wherein the lower alcohol is ethanol.

5. The pharmaceutical composition for inflammatory bowel disease according to claim 1, wherein the fraction is an organic solvent fraction.

6. The pharmaceutical composition for inflammatory bowel disease according to claim 4, wherein the organic solvent is at least one selected from the group consisting of chloroform, hexane, ethyl acetate and butanol.

7. The pharmaceutical composition for inflammatory bowel disease according to claim 1, wherein the fraction is a hexane fraction, a chloroform fraction, an ethyl acetate fraction or a butanol fraction, obtained by means of sequential fractionation with hexane, chloroform, ethyl acetate, butanol.

8. A health functional food comprising an Indigo Pulverata Levis extract or a fraction thereof as an effective ingredient for preventing or alleviating inflammatory bowel disease.

9. A method for preventing or treating inflammatory bowel disease, comprising a step of administering a pharmaceutical composition containing an Indigo Pulverata Levis extract or a fraction thereof as an effective ingredient into subjects in need.

10. Use of an Indigo Pulverata Levis extract or a fraction thereof for preventing or treating inflammatory bowel disease.

11. Use of an Indigo Pulverata Levis extract or a fraction thereof for producing a drug having an effect of preventing or treating inflammatory bowel disease.
